# EUROPÄISCHE PATENTSCHRIFT

(11) **EP 2 299 973 B1**
(45) Veröffentlichungstag und Bekanntmachung des Hinweises auf die Patenterteilung: **15.02.2017**
(21) Anmeldenummer: 09728128.1
(22) Anmeldetag: 01.04.2009
(51) Int. Cl.: A61K 9/08, A61K 31/05, A61K 31/715

(54) **PHARMAZEUTISCHE ZUBEREITUNG MIT PERMETHYLIERTEM CYCLODEXTRIN**
PHARMACEUTICAL PREPARATION COMPRISING PERMETHYLATED CYCLODEXTRIN
PRÉPARATION PHARMACEUTIQUE COMPRENANT DU CYCLODEXTRINE PERMETHYLÉ

(30) Priorität: 04.04.2008 EP 08006866
(43) Veröffentlichungstag der Anmeldung: 30.03.2011
(73) Patentinhaber: Roewer, Norbert, 97082 Würzburg (DE); Broscheit, Jens, 97209 Würzburg (DE)
(72) Erfinder: Roewer, Norbert, 97082 Würzburg (DE); Broscheit, Jens, 97209 Würzburg (DE)
(74) Vertreter: Glawe, Delfs, Moll
(86) Internationale Anmeldenummer: PCT/EP2009/002381
(87) Internationale Veröffentlichungsnummer: WO 2009/121585

(56) Entgegenhaltungen:
- EP-A- 0 214 647
- EP-A- 1 704 858
- WO-A-01/30391
- US-A1- 2007 093 448
- US-B1- 6 576 261
- US-B1- 6 699 849
- BRAGA S S ET AL: "Inclusion of molybdenocene dichloride (Cp2MoCl2) in 2-hydroxypropyl- and trimethyl-beta-cyclodextrin: Structural and biological properties" JOURNAL OF ORGANOMETALLIC CHEMISTRY, ELSEVIER-SEQUOIA S.A. LAUSANNE, CH, Bd. 690, Nr. 12, 15. Juni 2005 (2005-06-15), Seiten 2905-2912, XP004922460 ISSN: 0022-328X

## Beschreibung

Die Erfindung betrifft eine pharmazeutische Zubereitung, für die präoperative Anxiolyse oder für anästhetische Zwecke.

In der Pharmazie ist es ein häufiges Problem, einen pharmazeutischen Wirkstoff so zu formulieren, dass er mittels einer bestimmten Applikationsart in der gewünschten Konzentration und möglichst effizient an den vorgesehenen Wirkort gebracht wird. So muss ein für intravenöse Applikation vorgesehener Wirkstoff in gewissem Umfang wasserlöslich sein, damit überhaupt eine systemische Konzentration im Blut erreicht werden kann. Andererseits muss er in der Regel eine gewisse Lipophilie aufweisen, um am vorgesehenen Wirkort ggf. Zellmembranen durchdringen zu können. Ein lediglich gut wasserlöslicher Wirkstoff kann beispielsweise nach intravenöser Applikation eine hohe systemische Konzentration im Blut aufbauen, er wird aber beispielsweise bei zu geringer Lipophilie eine geringe Bioverfügbarkeit aufweisen, da am vorgesehenen Wirkort möglicherweise ein unzureichender Durchtritt durch die Zellmembran erfolgt.

Bei bestimmten Applikationsarten wie beispielsweise der transmucosalen Applikation kann die Bioverfügbarkeit eines Wirkstoffes darunter leiden, dass der Durchtritt durch die Mucosa zu langsam oder verzögert erfolgt und deswegen eine hinreichende systemische Konzentration des Wirkstoffes nicht oder zu langsam erreicht wird. Diese Problematik sei nachfolgend erläutert am Beispiel der sogenannten präoperativen Anxiolyse.

Vor einer Anästhesie und Operation eines Patienten wird regelmäßig eine Prämedikation durchgeführt. Deren vorrangiges Ziel ist die Stressreduktion bzw. -vermeidung durch eine sogenannte Anxiolyse. Die präoperative psychische Verfassung eines Patienten hat einschlägigen Untersuchungen zufolge erheblichen Einfluss auf das intraoperative Kreislaufverhalten und den postoperativen Schmerzmittelbedarf. Ungenügende Anxiolyse kann z.B. zu erhöhten Magensäuresekretion mit der Gefahr der Aspiration bei der Narkoseeinleitung führen. Dies kann lebensbedrohlich sein.

Zur Anxiolyse werden im Stand der Technik regelmäßig Benzodiazepine verwendet.

Die Prämedikation am Operationstag erfolgt in aller Regel peroral durch Einnahme eines schnell anflutenden Benzodiazepins, insbesondere Midazolam, etwa 45 bis 60 min vor Anästhesiebeginn.

Die Bioverfügbarkeit einer peroralen Anwendung ist sehr variabel und damit schwer kalkulierbar. Fehldosierungen mit gegebenenfalls unzureichender Anxiolyse sind nicht auszuschließen.

Ferner ist die bei peroraler Gabe nötige Anflutungszeit zur Erzielung eines ausreichenden Plasmaspiegels (45 bis 60 min) nicht mehr vereinbar mit den Anforderungen an das Zeitmanagement in Krankenhäusern. Zur Erzielung eines hohen Auslastungsgrades der Operationseinrichtungen erfolgt der Abruf eines Patienten zur Operation regelmäßig mit einem Vorlauf von lediglich 15 min. Dieser geringe Vorlauf erlaubt keine hinreichende Anxiolyse durch perorale Benzodiazepin Gabe.

Es ist bereits vorgeschlagen worden (WO-A-01/30391), Midazolam mit β-Cyclodextrin zu einer transmucosal anwendbaren Lösung zu formulieren.

US 6,699,849 B1 offenbart die Komplexierung verschiedener Benzodiazepine mit verschiedenen Cyclodextrinen, die sämtlich OH-Gruppen tragen, um die Wasserlöslichkeit zu verbessern.

Der Erfindung liegt die Aufgabe zu Grunde, eine pharmazeutische Zubereitung der eingangs genannten Art zu schaffen, die eine sichere und zuverlässige Applikation von Midazolam bei hinreichend zuverlässig vorhersagbaren Wirkprofil ermöglicht.

Erfindungsgemäß ist vorgesehen, dass sie enthält:
a) Midazolam;
b) permethyliertes β-Cyclodextrin mit einem Substitutionsgrad von 3 Methylgruppen pro Glucopyranoseeinheit;
   wobei permethyliertes β-Cyclodextrin und Midazolam einen Komplex bilden.

Im Stand der Technik (WO-A-01/30391) sind bereits Cyclodextrine als Hilfsstoffe vorgeschlagen worden, die pharmazeutische Wirkstoffe komplexieren, gegebenenfalls in wässriger Lösung stabilisieren bzw. löslich machen und die Membrangängigkeit erhöhen sollen.

Die vorliegende Erfindung hat erkannt, dass überraschender Weise spezielle Cyclodextrine, nämlich permethyliertes β-Cyclodextrin mit einem Substitutionsgrad von 3 Methylgruppen pro Glucopyranoseeinheit, einerseits eine sehr gute Stabilisierung und Komplexierung von Midazolam in wässriger Lösung ermöglicht und andererseits den Durchtritt des Wirkstoffs durch Membranen erleichtet.

Cyclodextrine weisen generell eine Toroidform auf und besitzen eine entsprechend geformte Kavität. Die erfindungsgemäß verwendeten permethylierten Cyclodextrine weisen eine Reihe von vorteilhaften Eigenschaften auf, die einerseits die Komplexierung und andererseits die gezielte Freisetzung am Wirkort des in Anspruch 1 näher definierten pharmazeutischen Wirkstoffs begünstigen. Die aufgrund der Permethylierung vollständige Abwesenheit von OH-Gruppen begünstigt den Eintritt hydrophober pharmazeutischer Wirkstoffe (aromatische Gruppe bzw. aromatischer Teil) in die Kavität zwecks Komplexierung. Bei nicht vollständiger Methylierung können die Eingänge bzw. Ränder der Kavität durch noch vorhandene OH-Gruppen am Cyclodextrin hydrophil sein und so die Komplexierung hydrophober Wirkstoffe erschweren.

Die Erfindung hat ferner erkannt, dass bei nicht vollständiger Methylierung vorhandene Rest-OH-Gruppen zu einer unerwünschten Aggregierung bzw. Agglomerierung von Cyclodextrinmolekülen in wässriger Lösung durch Bildung von Wasserstoffbrückenbindungen führen kann. Die Verwendung permethylierten β-Cyclodextrins vermeidet dies.

Erfindungsgemäß wird ein wasserlöslicher und in wässriger Lösung stabiler, nicht aggregierter/agglomerierter Komplex gebildet, der Midazolam schnell und zuverlässig am Wirkort zur Verfügung stellen kann. Eine mögliche Erklärung für die überraschend verbesserte Wirkung des permethylierten β-Cyclodextrins könnte sein, dass permethylierte Cyclodextrine einen erhöhten lipophilen Charakter aufweisen und daher die Wechselwirkung mit der Membran und damit die Resorption des vom Cyclodextrin komplexierten Wirkstoffes erleichtern.

Die hohe Lipophilie des erfindungsgemäß verwendeten β-Cyclodextrins drückt sich in einem hohen logP-Wert aus, der den Verteilungskoeffizienten der entsprechenden Substanz in dem Lösemittelsystem Octanol/Wasser beschreibt. Dieser logP-Wert liegt erfindungsgemäß bei 9.

β-Cyclodextrin weist sieben Glucopyranoseeinheiten auf. Jede Glucopyranoseeinheit weist drei OH-Gruppen auf, die substituiert, im vorliegenden Fall methyliert werden können. Permethyliertes β-Cyclodextrin ist vollständig substituiert, das heißt der Substitutionsgrad beträgt 3.

Die erfindungsgemäß verwendeten Cyclodextrine haben eine polare Außenfläche und eine hydrophobe Kavität (Hohlraum) im Inneren. Sie komplexieren den bevorzugt lipophilen pharmazeutischen Wirkstoff und machen ihn auf diese Weise in wässriger Phase löslich. Am pharmakologischen Wirkort setzt der Komplex den Wirkstoff so frei (möglicherweise durch ein "Andocken" des Cyclodextrins an die Membran), so dass die Membran ohne weiteres durchdrungen werden kann.

Die Aufnahme von Arzneimitteln in Zielzellen erfolgt häufig passiv, da hierfür normalerweise keine Transportsysteme über die Zellmembran zur Verfügung stehen. Der Grad des passiven Diffusionsflusses eines Arzneimittels durch eine biologische Membran wird wesentlich durch die Lipophilie des Arzneimittels sowie dessen Konzentrationsgradient an der Membran bestimmt. Diese beiden Bedingungen sind allerdings häufig gegenläufig. Hohe Lipophilie (und damit eine gute Fähigkeit zur passiven Durchdringung der Zellmembran am Wirkort) bedingt häufig eine geringe Wasserlöslichkeit, so dass kein großer Konzentrationsgradient über der als Barriere wirkenden Membran erzeugt werden kann, da das entsprechende Arzneimittel in der wässrigen Phase außerhalb der Zellmembran wenig löslich ist.

Bei der erfindungsgemäßen pharmazeutischen Zubereitung ist der pharmazeutische Wirkstoff Midazolam durch das Cyclodextrin so komplexiert, dass er als Komplex gut wasserlöslich ist und damit in höherer Konzentration an die Membran gebracht werden kann. Einmal an die Membran herantransportiert, wird er offensichtlich in einer Art und Weise freigesetzt, dass die lipophilen Eigenschaften beim Durchdringen der Membran dominieren. Bei der erfindungsgemäßen Zubereitung erlaubt also der Komplex aufgrund seiner Wasserlöslichkeit die Einstellung eines hohen Konzentrationsgefälles über die Membranbarriere, gleichzeitig kann die Lipophilie des pharmazeutischen Wirkstoffes eine hohe Durchdringungsgeschwindigkeit der Membranbarriere bewirken. Die beiden im Stand der Technik in der Regel gegenläufigen Faktoren für eine gute Durchdringung einer Zellmembran am pharmakologischen Wirkort können erfindungsgemäß also in überraschender Weise synergistisch kombiniert werden.

Der pH-Wert der erfindungsgemäßen pharmazeutischen Zubereitung liegt vorzugsweise zwischen 4 und 7, besonders bevorzugt zwischen 5,5 und 6,5. Dies entspricht dem pH-Wert der Schleimhäute, so dass eine beispielsweise eine transmucosale Applikation ohne Irritationen oder sonstige unangenehme Nebenwirkungen möglich ist.

Permethyliertes β-Cyclodextrin besitzt einen zur Aufnahme eines pharmazeutischen Wirkstoffs geeigneten Hohlraum mit einem Durchmesser von etwa 0,6 bis 0,65 nm und einer Höhe von etwa 2 nm.

Wie oben beschrieben eignet sich eine erfindungsgemäße Zubereitung somit dafür, die Bioverfügbarkeit eines pharmazeutischen Wirkstoffes am vorgesehenen Wirkort zu erhöhen. Der Komplex der Zubereitung gelangt bei dieser Wirkweise (beispielsweise durch die Blutbahn) an den pharmazeutischen Wirkort, dort wird der Wirkstoff aus dem Komplex freigesetzt und durchdringt die Zellmembran. Das Cyclodextrin selbst kann die Zellmembran nicht durchdringen.

Gemäß einem weiteren Aspekt der Erfindung kann die erfindungsgemäße Zubereitung nicht nur verwendet werden, um die Bioverfügbarkeit durch Transport des Wirkstoffes (mittels des Cyclodextrinkomplexes) durch die Blutbahn an den Wirkort sicherzustellen, sondern um bei bestimmten Applikationsarten überhaupt erst die Einstellung einer hinreichend großen und im zeitlichen Profil hinreichend sicher vorhersagbaren systemischen Wirkstoffkonzentration zu ermöglichen. Bei dieser Variante der Erfindung setzt der Komplex der erfindungsgemäßen Zubereitung den Wirkstoff nicht am eigentlichen Wirkort frei, sondern verbessert beispielsweise bei einer transmucosalen Applikation den Durchtritt des Wirkstoffes durch die Mucosamembran, so dass schnell und vorhersagbar eine bestimmte systemische Wirkstoffkonzentration aufgebaut wird. Bei dieser Variante der Erfindung erfolgt somit die Freisetzung des Wirkstoffes aus dem Cyclodextrinkomplex bereits beim Durchtritt durch die Mucosamembran.

Die erfindungsgemäße Zubereitung eignet sich somit auch für eine Formulierung zur transmucosalen Applikation.

Eine pharmazeutische Zubereitung zur transmucosalen Applikation umfasst einen mit entsprechenden Hilfsstoffen formulierten pharmazeutischen Wirkstoff (Midazolam)der ganz oder zu wesentlichen Teilen durch Schleimhäute (insbesondere Nasen- und/oder Mundschleimhäute) systemisch aufgenommen wird. Die transmucosale Applikation hat den grundsätzlichen Vorteil, dass sie eine schnellere, zuverlässigere und besser kalkulierbare systemische Anflutung eines Wirkstoffes ermöglicht, da die Einstellung eines entsprechenden Serumspiegels nicht von den Besonderheiten und Unwägbarkeiten des Magen-Darm-Traktes abhängt. (First-Pass Effekt bedingt durch die Leberpassage nach enteraler Resorption). Die erfindungsgemäße Zubereitung kann insbesondere für eine transnasale, intralinguale, intestinale oder orovestibulare Applikation formuliert sein. Die intestinale Resorption kann durch diese Zubereitung auch profitieren.

Der Anteil des Midazolams an der pharmazeutischen Zubereitung kann bei einer bevorzugten Ausführungsform 0,5 bis 2 Gew.-%, insbesondere 1 Gew.-% betragen. Die Zubereitung ist bevorzugt eine wässrige Lösung.

Gegenstand der Erfindung ist ferner die Verwendung eines permethylierten β-Cyclodextrins mit einem Substitutionsgrad von 3 Methylgruppen pro Glucopyranoseeinheit zur Herstellung einer pharmazeutischen Zubereitung, beispielsweise zur transmucosalen Applikation. Bevorzugt ist ferner die erfindungsgemäße Verwendung zur Herstellung eines Medikaments zur präoperativen Anxiolyse.

Gemäß einem weiteren Aspekt der Erfindung kann eine erfindungsgemäße pharmazeutische Zubereitung verwendet werden, um einen nicht oder kaum wasserlöslichen Wirkstoff einer intravenösen Administration zugänglich zu machen. Dies sei am Beispiel von Propofol erläutert.

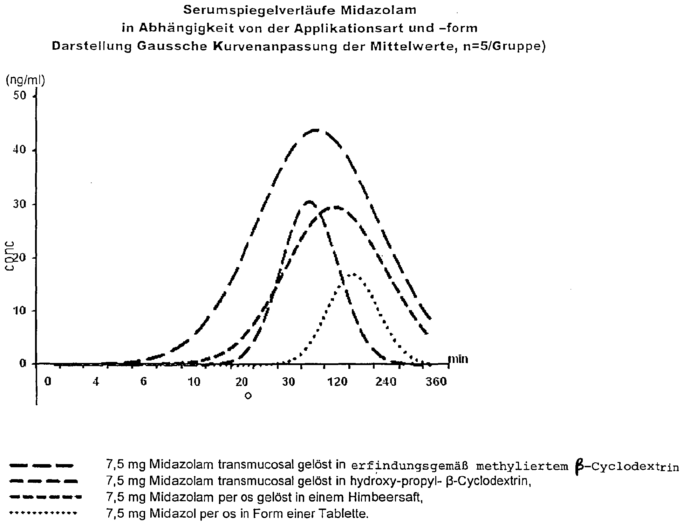

Ausführungsbeispiele der Erfindung werden nachfolgend beschrieben. Die Zeichnung zeigt den Serumspiegelverlauf von Midazolam für die beschriebene Ausführungsform der Erfindung und Vergleichsbeispiele.

### Beispiel 1

### Midazolam-Zubereitung für transmucosale Applikation

Eine 1%ige Midazolam-Zubereitung für transnasale Applikation wird wie folgt formuliert:

| | |
|---|---|
| Midazolam: | 10 mg |
| Permethyliertes β-Cyclodextrin (Methylierungsgrad:3) | 150 mg |
| Hypromellose 400: | 1 mg |
| H₃PO₄, konz.: | 2,6 mg |
| NaOH 10%: | q.s. Zur Herstellung |
| Kaliumsorbat: | eines ph-Wertes von 4,2 1,4 mg |
| Wasser: | ad 1 ml. |

Hypromellose 400 ist eine Hydroxypropylmethyl-Cellulose mit einem Molekulargewicht von etwa 400. Sie dient als Benetzungsmittel für die Schleimhäute. Der pH-Wert der so hergestellten Lösung beträgt 4,2.

Im vorliegenden Beispiel beträgt das Massenverhältnis zwischen dem Cyclodextrin und dem pharmazeutischen Wirkstoff 15:1. Im Rahmen der Erfindung sind allgemein Verhältnisse zwischen Cyclodextrin und pharmazeutischen Wirkstoff von 50:1 bis 10:1, weiter 40:1 bis 12:1, weiter 20:1 bis 12:1 bevorzugt.

Jeweils 0,75 ml dieser Lösung, (enthaltend 7,5 mg Midazolam) wurden einer Gruppe von fünf Probanden transmucosal appliziert.

Zum Vergleich mit dem Stand der Technik wurde drei weiteren Gruppen von jeweils fünf Probanden die gleiche Wirkstoffmenge von 7,5 mg Midazolam in folgender Weise appliziert:
**Vergleichsbeispiel 1:** Das permethylierte-β-Cyclodextrin des erfindungsgemäßen Beispiels wurde ersetzt durch Hydroxypropyl-β-Cyclodextrin.
**Vergleichsbeispiel 2:** 7,5 mg Midazolam per os gelöst in einem Himbeersaft.
**Vergleichsbeispiel 3:** 7,5 mg Midazolam per os in Form einer Tablette.

Bei dem Vergleichsbeispiel 1 wurde mit Ausnahme des Austauschs der Cyclodextrine die gleiche Rezeptur verwendet wie im Beispiel 1.

Die Mittelwerte der Serumspiegelverläufe von Midazolam als Funktion der Zeit nach der entsprechenden Applikation sind in der Figur dargestellt. Man erkennt, dass die erfindungsgemäße Zubereitung die schnellste Anflutung kombiniert mit der Erreichung des höchsten Serumspiegels und der längsten Wirkdauer.

Eingangs ist beschrieben worden, dass in Krankenhäusern häufig der Abruf von Patienten zu einer Operation mit einem Vorlauf von lediglich 15 min. erfolgt. Somit steht lediglich dieses Zeitfenster für eine anxiolytische Prämedikation zur Verfügung, da man ein schnell wirkendes Benzodiazepin wie Midazolam nicht einfach auf Verdacht geben kann, ohne zu wissen, wann die Operation tatsächlich stattfinden wird.

Die Zeichnung zeigt, dass lediglich die erfindungsgemäße Formulierung bereits nach 15 min. einen nennenswerten Serumspiegel aufgebaut hat, der bereits eine anxiolytische Wirkung hat. Sämtliche Applikationsformen des Standes der Technik (einschließlich der transmucosalen Applikation mit Hydroxypropyl-β-Cyclodextrin als Komplexbildner) fängt nach 15 min. überhaupt erst an, einen Serumspiegel aufzubauen. In der Praxis bedeutet dies, dass eine anxiolytische Prämetikation des Standes der Technik nach 15 min. praktisch noch nicht wirkt und damit ihren Zweck vollständig verfehlt.

Zum Vergleich mit dem Stand der Technik wurde drei weiteren Gruppen von jeweils fünf Probanden die gleiche Wirkstoffmenge von 7,5 mg Midazolam in folgender Weise appliziert:
**Vergleichsbeispiel 1:** Das permethylierte-β-Cyclodextrin des erfindungsgemäßen Beispiels wurde ersetzt durch Hydroxypropyl-β-Cyclodextrin.
**Vergleichsbeispiel 2:** 7,5 mg Midazolam per os gelöst in einem Himbeersaft.
**Vergleichsbeispiel 3:** 7,5 mg Midazolam per os in Form einer Tablette.

Bei dem Vergleichsbeispiel 1 wurde mit Ausnahme des Austauschs der Cyclodextrine die gleiche Rezeptur verwendet wie im Beispiel 1.

Die Mittelwerte der Serumspiegelverläufe von Midazolam als Funktion der Zeit nach der entsprechenden Applikation sind in der Figur dargestellt. Man erkennt, dass die erfindungsgemäße Zubereitung die schnellste Anflutung kombiniert mit der Erreichung des höchsten Serumspiegels und der längsten Wirkdauer.

Eingangs ist beschrieben worden, dass in Krankenhäusern häufig der Abruf von Patienten zu einer Operation mit einem Vorlauf von lediglich 15 min. erfolgt. Somit steht lediglich dieses Zeitfenster für eine anxiolytische Prämedikation zur Verfügung, da man ein schnell wirkendes Benzodiazepin wie Midazolam nicht einfach auf Verdacht geben kann, ohne zu wissen, wann die Operation tatsächlich stattfinden wird.

Die Zeichnung zeigt, dass lediglich die erfindungsgemäße Formulierung bereits nach 15 min. einen nennenswerten Serumspiegel aufgebaut hat, der bereits eine anxiolytische Wirkung hat. Sämtliche Applikationsformen des Standes der Technik (einschließlich der transmucosalen Applikation mit Hydroxypropyl-β-Cyclodextrin als Komplexbildner) fängt nach 15 min. überhaupt erst an, einen Serumspiegel aufzubauen. In der Praxis bedeutet dies, dass eine anxiolytische Prämetikation des Standes der Technik nach 15 min. praktisch noch nicht wirkt und damit ihren Zweck vollständig verfehlt.

### Beispiel 2

### Propofol-Zubereitung für i.v. Applikation

Es werden zwei 1%ige Propofol Formulierungen zur Verfügung gestellt. Als Formulierung des Standes der Technik wurde eine unter dem Handelsnamen Diprivan® erhältliche Lipidemulsion zur Verfügung gestellt, die 1% Propofol in einer Emulsionsgrundlage aus Sojaöl, Glycerin und Eiphosphatid enthält.

Erfindungsgemäße Formulierung:

| | |
|---|---|
| Propofol: | 10 mg |
| Permethyliertes β-Cyclodextrin : | 80,2 mg |
| H₂PO₄, konz. : | 2,6 mg |
| NaOH 10%: | q.s. zur Einstellung |
| Wasser: | eines pH-Wertes von 6,8 ad 1 ml |

Diese Formulierung wird nachfolgend als CD-Propofol bezeichnet.

Je 12 Göttinger Mini-Schweine mit einem Körpergewicht zwischen 48 und 53 kg wurden randomisiert mit Diprivan® und CD-Propofol anästhesiert. Die Administration erfolgte mittels eines peripheren Venenkatheters.

Die Überwachung der Narkosetiefe erfolgte durch Cerebral State Monitoring (CSM) und entsprechende Aufzeichnung des Cerebral State Index (CSI). Als erwünschte Narkosetiefe wurde ein CSI von 40 bis 60 eingestellt. Die Überwachung der Narkose erfolgte ferner durch Aufnahme eines Elektrokardiogramms (ECG), Pulsoxymetrie (Ermittlung des SaO₂-Wertes und Capnometrie (Ermittlung des pCO₂-Wertes.

Die equipotenten Dosen zur Erzielung der o. g. Narkosetiefe betrugen 87,5 ± 12,5 mg bei der erfindungsgemäßen Formulierung gegenüber 225 ± 12,5 mg bei der Formulierung des Standes der Technik (Diprivan).

Die erfindungsgemäße Formulierung zeigt eine wesentlich schnellere An- und Abflutung. Die Wirkung der erfindungsgemäßen Formulierung setzt 1 min nach der Administration ein, bei der Formulierung des Standes der Technik beträgt dieser Zeitraum 2 min. Das Wirkungsende tritt bei der erfindungsgemäßen Formulierung nach 19 min ein, bei der Formulierung des Standes der Technik nach 25,5 min. Die erfindungsgemäße Formulierung bewirkt im Gegensatz zum Stand der Technik keinerlei Emulyse.

## Patentansprüche

1. Pharmazeutische Zubereitung zur Applikation eines pharmazeutischen Wirkstoffs, **dadurch gekennzeichnet, dass** sie enthält:
a) Midazolam;
b) permethyliertes β-Cyclodextrin mit einem Substitutionsgrad von 3 Methylgruppen pro Glucopyranoseeinheit;
wobei permethyliertes β-Cyclodextrin und Midazolam einen Komplex bilden.

2. Pharmazeutische Zubereitung nach Anspruch 1, **dadurch gekennzeichnet, dass** sie einen pH-Wert von 4 bis 7, vorzugsweise 5,5 bis 6,5 aufweist.

3. Pharmazeutische Zubereitung nach Anspruch 1 oder 2, **dadurch gekennzeichnet, dass** sie für eine transmucosale Applikation formuliert ist.

4. Pharmazeutische Zubereitung nach Anspruch 3, **dadurch gekennzeichnet, dass** sie für eine transnasale, intralinguale, intestinale oder orovestibulare Applikation formuliert ist.

5. Pharmazeutische Zubereitung nach Anspruch 1 bis 4, **dadurch gekennzeichnet, dass** der Gehalt des Midazolams 0,5 bis 2 Gew.-% beträgt.

6. Pharmazeutische Zubereitung nach einem der Ansprüche 1 bis 5, **dadurch gekennzeichnet, dass** sie eine Formulierung eines Anästhetikums für i.v.-Applikation ist.

7. Pharmazeutische Zubereitung nach einem der Ansprüche 1 bis 6 zur Herstellung eines Medikaments zur präoperativen Anxiolyse oder eines Anästhetikums für i.v. Applikation.

## Claims

1. Pharmaceutical preparation for the application of a pharmaceutical active substance, **characterized in that** it contains:
a) midazolam;
b) permethylated β-cyclodextrin with a degree of substitution of 3 methyl groups per glucopyranose unit;
where the permethylated β-cyclodextrin and the midazolam form a complex.

2. Pharmaceutical preparation according to Claim 1, **characterized in that** it has a pH from 4 to 7, preferably from 5.5 to 6.5.

3. Pharmaceutical preparation according to Claim 1 or 2, **characterized in that** it is formulated for transmucosal application.

4. Pharmaceutical preparation according to Claim 3, **characterized in that** it is formulated for transnasal, intralingual, intestinal or orovestibular application.

5. Pharmaceutical preparation according to Claim 1 to 4, **characterized in that** the content of the midazolam is 0.5 to 2 wt.%.

6. Pharmaceutical preparation according to one of Claims 1 to 5, **characterized in that** it is a formulation of an anesthetic for i.v. application.

7. Pharmaceutical preparation according to one of Claims 1 to 6 for the production of a medicinal product for preoperative anxiolysis or an anesthetic for i.v. application.

## Revendications

1. Préparation pharmaceutique pour l'application d'un principe actif pharmaceutique, **caractérisée en ce qu'**elle contient :
a) du midazolam ;
b) de la β-cyclodextrine perméthylée avec un taux de substitution de 3 groupes méthyles par unité de glucopyranose ;
la β-cyclodextrine perméthylée et le midazolam formant un complexe.

2. Préparation pharmaceutique selon la revendication 1, **caractérisée en ce qu'**elle présente une valeur de pH de 4 à 7, de préférence de 5,5 à 6,5.

3. Préparation pharmaceutique selon la revendication 1 ou 2, **caractérisée en ce qu'**elle est formulée pour une application transmucosale.

4. Préparation pharmaceutique selon la revendication 3, **caractérisée en ce qu'**elle est formulée pour une application transnasale, intraliguale, intestinale ou orovestibulaire.

5. Préparation pharmaceutique selon la revendication 1 à 4, **caractérisée en ce que** la teneur en midazolam est de 0,5 à 2 % en poids.

6. Préparation pharmaceutique selon l'une des revendications 1 à 5, **caractérisée en ce qu'**il s'agit d'une formulation d'anesthésique destiné à une application i.v.

7. Préparation pharmaceutique selon l'une des revendications 1 à 6, pour la fabrication d'un médicament pour une anxiolyse pré-opératoire ou d'un anesthésique destiné à une application i.v.
